Europäisches Patentamt

European Patent Office

Office européen des brevets

(11) Publication number: **0 031 519**
A1

# (12) EUROPEAN PATENT APPLICATION

(21) Application number: **80107797.5**

(22) Date of filing: **11.12.80**

(51) Int. Cl.³: **C 07 B 9/00,** B 01 J 12/00,
B 01 J 19/24
// C07C19/08, C07C49/167,
C07C23/08, C07C43/12,
C07C53/48, C07C85/24

(30) Priority: **26.12.79 US 107124**
**26.12.79 US 107123**

(43) Date of publication of application: **08.07.81**
**Bulletin 81/27**

(84) Designated Contracting States: **DE FR GB IT**

(71) Applicant: **ALLIED CHEMICAL CORPORATION,
Columbia Road and Park Avenue P.O. Box 2245R (Law
Dept.), Morristown New Jersey 07960 (US)**

(72) Inventor: **Nychka, Henry Robert, 180 Hillcrest Road, East
Aurora New York 14052 (US)**
Inventor: **Hino, John Bernard, 92 Southgate Road,
Cheektowaga New York 14215 (US)**
Inventor: **Eibeck, Richard Elmer, 23 Pine Terrace,
Orchard Park New York 14127 (US)**
Inventor: **Robinson, Martin Alvin, 167 Wood Acres Drive,
East Amherst New York 14051 (US)**

(74) Representative: **Collier, Jeremy Austin Grey et al,
J.A.Kemp & Co. 14, South Square Gray's Inn, London
WC1R 5EU (GB)**

(54) **Preparation of fluorinated organic compounds with elemental fluorine and fused alumina reactor.**

(57) Highly fluorinated compounds are prepared by reacting fluorine with an organic compound having at least one fluorinatable carbon using a fused alumina porous tube. The fluorine is introduced into the porous tube and migrates outwardly. The organic is introduced outside the porous tube. A diluent such as sulfur hexafluoride or nitrogen may be used, but is not required. The process is particularly applicable to the production of octafluoropropane.

## DESCRIPTION

### PREPARATION OF FLUORINATED ORGANIC COMPOUNDS WITH ELEMENTAL FLUORINE AND FUSED ALUMINA POROUS REACTOR

### BACKGROUND OF THE INVENTION

Present invention relates to the production of fluorinated organic compounds and especially compounds having the relatively high number of fluorines per carbon.

The production of highly fluorinated compounds has been conducted by a variety of techniques. For many compounds, however, conventional reactions and especially those which employ HF as the fluorinating source, provide insufficient levels of fluorination. In addition, when elemental fluorine is used, many compounds are either converted to carbon as a by-product or cleaved if more than one carbon is initially present. Futhermore, when hetero atoms such as the oxygen of acetone, the oxygen of ethers or the nitrogen of poly-alkylamines are present, fluorine causes side reactions such as cleavage or polymerization of the starting material.

In a copending application (U.S. Application 107,122, filed December 26, 1979) a method is described of reacting organic compounds having a fluorinatable carbon and at least two carbons with fluorine in a porous tube. In that application it is indicated that, using the metal porous tubes employed therein, it was necessary to use perfluorinated diluents such as sulfur hexafluoride in order to prevent cleavage. While such reactions give the desired product in high yields, the introduction of such a diluent can complicate recovery of the product.

In addition disclosures of Kurtz, including Canadian Patent 990,738 and commonly assigned copending U.S. Patent Application 644,788 filed December 29, 1975 (now U.S. Patent 4,187,253 issued February 5, 1980) disclose porous tubes, including both metal tubes and fused alumina tubes, for the chlorination of organic compounds. While the above-described patents indicate the suitability of such metal tubes for fluorination, it was previously thought that fused alumina would be unsuitable for fluorinations because of the usually rapid reaction of alumina with fluorine to produce aluminum fluoride. Because aluminum fluoride is less dense than alumina, it would be expected that the pores of the tube would be clogged by expansion if fluorine were introduced into the porous tube.

The present invention also relates to the production of perfluoropropane or octafluoropropane which is known to be useful in plasma etching of electronic components and is a dielectric gas.

The production of perfluoropropane has been attempted from a variety of starting materials, but no process has been developed which produces the material in high yields without a multitude of steps. Exemplary starting materials are propane, propene, hexafluoropropene and partially halogenated propanes and propenes.

U.S. Patent 4,158,023 to von Halasz describes the production of perfluoropropane from hexafluoropropene in two steps. This reference indicates that the direct conversion with elemental fluorine results in poor yields. Since hexafluoropropene is itself fairly hard to produce in good yields, the overall process becomes involved with a multitude of steps and expense.

U.S. Patent 3,840,445 to Paul et al. describes electrochemical fluorination of propane to perfluoropropane. The reference indicates the difficulty in separating propane from the product by distillation, and, accordingly, employs a two-step process of electrochemical fluorination. Although the product need be separated only from partially fluorinated propane when the two steps are used, the process thereby becomes more involved, and the potential for overall yield losses increases.

The production of perfluoropropane from propane by elemental fluorination in a jet reactor has been described in an article by A. F. Maxwell et al. in _Journal of the American Chemical Society_, Vol. 52, pp. 5827-30 (November 20, 1960). The tables in this article report production of perfluoropropane which suggests a yield, in the best case, of about 50 percent based on propane fed.

## BRIEF DESCRIPTION OF THE INVENTION

It has been surprisingly found that by using a fused alumina porous tube, fluorine can be introduced into the inner zone and organics introduced outside the porous tube, with the result being neither reaction of fluorine with the tube nor the cleavage and/or polymerization reactions that might be expected. Instead, the use of a fused alumina tube permits the reaction to be conducted in conventional diluents such as nitrogen or helium or even without diluents; and, furthermore, even with a perfluorinated diluent, higher yields of the desired highly fluorinated compounds are achieved than with metal porous tubes.

Thus the present invention includes a method of preparing fluorinated organic compounds which comprises:

(a) introducing a first gaseous reactant stream comprising fluorine into an elongated inner zone surrounded by an aluminum oxide porous member fused at sufficiently high temperature to be resistant to fluorine corrosion; and

(b) introducing a second gaseous reactant stream outside the aluminum oxide porous member comprising at least one organic compound with at least one fluorinatable carbon selected from the group consisting of carbons covalently bonded to H, Cl or Br and carbons olefinically or acetylenically bonded to other carbons;

the pressure in the elongated inner zone being greater than the pressure outside the aluminum oxide porous member.

A preferred form of the present invention involves the production of octafluoropropane (also called perfluoropropane) from three carbon organic compounds, with the octafluoropropane recovered from the effluent outside the porous tube.

## DETAILED DESCRIPTION OF THE INVENTION

The present invention employs a porous fused aluminum oxide (alumina) tube for the elemental fluorination of organic compounds. When using such a tube for fluorination, unlike the fluorinations of copending U.S. application 107,122 in a porous metal tube, but like the chlorinations described in Canadian Patent 990,738 and U.S. Application 644,788, it is sufficient to employ common inert diluents such as nitrogen and noble gases and still achieve good yields with low losses to cleavage reactions or carbon formation. A perfluorinated diluent such as sulfur hexafluoride $CF_4$, $C_2F_6$ or $C_3F_8$ (which may also be the reaction product) may also be employed as the diluent. Alternatively, HF, the reaction byproduct, may be used as a diluent. Preferred diluents include nitrogen, sulfur hexafluoride and the fluorinated products (being recycled) or HF (also being recycled).

In addition, with porous alumina tubes, fluorination reactions can be conducted with neither the fluorine fed into the interior zone nor the organic fed outside the porous tube being diluted.

To minimize volumes and separation steps, it is preferred to use volume ratios of diluent to total reactants (including both fluorine and any organic having at least one fluorinatable carbon) of between 0:1 and about 5:1, preferably 0:1 to about 2:1, more preferably 0:1 to about 0.5:1 and most preferably 0:1 to about 0.1:1. While these low diluent feeds are preferred, some organics such as acetone are more stable to undesired byproduct formation if the reaction temperature is kept from becoming excessive, and employing some diluent is one means of reducing reaction temperature. If a diluent is used, it can be mixed with either reactant or both.

The organic compound used as a second gaseous reactant can have one or more carbons, at least one of which is "fluorinatable," by which is meant having an H, Cl or Br attached that can be replaced by F or an olefinic or acetylenic bond to another carbon that can be added across by fluorine. Acyclic and cycloaliphatc compounds are preferred over aromatic compounds. Compounds having at least one C-H bond are preferred over compounds having only fluorinatable carbons other than carbons bonded to hydrogen. While carbons bonded to "hetero" atoms such as Br, Cl or O are eventually fluorinated, the present process can also be made selective by using limited amounts of fluorine and/or mild conditions so as to preserve hetero atoms and replace only hydrogens and unsaturations by fluorine.

One preferred group of second reactants are the aliphatic hydrocarbons of one to eight carbons, including aliphatic hydrocarbons such as ethane, propane, butane and higher members of the series and olefinic hydrocarbons such as ethene, propene, butene and higher members of the series. These materials can be converted to the corresponding perfluoroalkanes of the formula $C_nF_{2n+2}$, or less fluorinated members having unsaturations or hydrogens, and less than 2n+2 F's. Two preferred reactants in this class are propane and propene, with the product being preferably perfluoropropane.

Another preferred group of second reactants are partially halogenated compounds of the above group having some or all of the hydrogens or unsaturations replaced by Cl, Br or F, but not all by F. Only hydrogens and unsaturations of such reactants are replaced by fluorine under mild conditions, especially temperature, and when the amount of fluorine is limited to that required for the desired fluorination. Thus $C_2H_5Cl$ can be converted to $C_2ClF_5$ with low temperatures and only five moles of fluorine per $C_2H_5Cl$; or it can be converted to $C_2F_6$ with six or more moles of fluorine per $C_2H_5Cl$. A preferred reactant in this class is hexafluoropropene.

Another preferred group of reactants are the ketones of 3-8 carbons such as acetone, methyl ethyl ketone and higher members of the series, together with partially halogenated ketones, as described above. In general hydrogens will first be replaced by fluorine, then Br, then Cl and only then the carbonyl oxygen. Preferred is acetone and partially halogenated acetone.

Another preferred group of second gaseous reactants are the cyclized form of any of the above such as cyclopentane, cyclohexane, and cyclohexene. In General, aromatics are not preferred since they are more likely to result in byproducts which can clog the pores of the porous tube.

The amount of fluorine introduced is preferably between about 50% and about 150% of the fluorine required to convert the second reactant to a perfluorinated product. Thus one $F_2$ is required for each C-H bond, one $F_2$ for each C=C bond and, generally, one $F_2$ for each hetero atom (such as Cl or Br) that it is desired to replace.

Thus, a mole of an alkane of the formula $C_nH_{2n+2}$ has a stoichiometric requirement of $2n + 2$ moles of fluorine and the preferred molar (or volume) feed of fluorine is between about $n + 1$ and about $3n + 3$ moles of alkane or a ratio of betwen about 5 and about 15 for propane, between about 4 and about 12 for ethane and between about 3 and about 9 for ethene. More preferred are amounts at the lower end of this range, up to a 10% excess over the stoichiometric amount.

For ketones, while between 50% and 150% of stoichiometric can be used, it is preferred to operate with excess organic, stoichiometric amounts or a slight excess of fluorine. Thus, for acetone, the preferred fluorine:acetone ratio (by volume or moles) is between about 1:1 and about 8:1; the more preferred ratio is between about 3:1 and about 6:1, and the most preferred ratio is between about 5:1 and about 6:1.

The ratio of fluorine to three carbon compound depends, in part, upon the particular three carbon compound employed. Thus, in general, one can compute the stoichiometric amount of fluorine required to convert the entire second reactant to a perfluoropropane as a first estimate of the desired amount of fluorine to be introduced.

For three carbon compounds having a hydrogen, one mole of fluorine is required (to make octafluoropropane) for each mole of hydrogen bonded to carbon such that half of the fluorine molecule can replace the hydrogen to form a C-F bond and the other half of the fluorine molecule can form HF by-product. For each unsaturation, one mole of fluorine is required to add across a double bond and to form two C-F bonds. For each other halogen present such as Cl or Br, or any other hetero atom present, one molecule of fluorine is generally required to both replace the hetero atom and form a by-product between fluorine and the hetero atom. In general, however, hetero atoms are preferably not present such that the three carbon compound has no elements other than carbon, hydrogen and fluorine.

While any amount of fluorine may be used, it is preferred to use between 50 and 150 percent of the stoichiometric amount. Thus, in the case of propane, 8 moles of fluorine per propane constitutes the stoichiometric amount, and a generally suitable range of fluorine is between about 4 and about 12 moles of fluorine per propane. Preferably, between about 5 and about 10 moles of fluorine are introduced into the inner zone per mole of propane introduced outside the elongated porous member. More preferably between about 8 and about 9.5 moles of fluorine are introduced per mole of fluorine.

In the case of propene, the stoichiometric amount is 7 moles of fluorine per propene. A generally suitable overall range is between about 3.5 and about 10.5 moles of fluorine per mole of propene. A preferred range of fluorine to propene ratios is between about 4:1 and about 8:1, more preferably between about 7:1 and about 8:1.

In the case of hexafluoropropene, the stoichiometric mole ratio is 1:1 while a range of about 0.5:1 to about 1.5:1 is suitable, as well as amounts higher, and especially lower in fluorine than that range, the preferred ratios are between about 0.7:1 and about 1.1:1, especially about 1:1.

Suitable and preferred ratios for other organics can be easily determined by routine experimentation after considering that less stable structures are subject to cleavage and may require avoiding large excesses of fluorine over the stoichiometric amount, while operation far below the stoichiometric amount may necessitate excessive recycling if highly fluorinated products are desired.

The porous alumina tube used in the present process is of a composition high in $Al_2O_3$ and preferably low in other materials such as silica and iron oxides. It is generally prepared at elevated temperatures from bauxite or "Bayer hydrate". It is normally porous when prepared due to the elimination in the vapor phase of water, carbon dioxide or other volatile components of the starting mineral. The primary criteria for selecting alumina materials is that they be resistant to corrosion by fluorine, which can convert most aluminas that are not fused to aluminum fluoride. If a porous alumina were so converted, the lesser density of aluminum fluoride compared to alumina would cause expansion so as to plug the pores. Thus a porous alumina can be treated with fluorine alone first to test for suitability, and such prior treatment appears desirable for any fresh porous tube before introducing the organic reactant. Pore size is not particularly critical.

A suitable porous aluminum oxide is sold by Norton Company of Worcester, Massachusetts as ALUNDUM (their registered trademark) AN-299A. Its properties are given below in Example 1.

Exact contact times and temperatures are not critical, with a suitable minimum temperature being the boiling point (under reaction pressures) of the least volatile reactant and a suitable maximum is 300°C for most organic reactants. A preferred maximum is about 250°C for more stable organics such as alkanes and about 200°C for less stable organics such as ketones (e.g. acetone). The maximum temperature on the surface of the porous tube can be adjusted by alteration of various flow rates including the flow of diluent, if any. Contact times are not critical, and may vary between about 1 and about 200 seconds, or higher or lower. Preferred contact times vary between about 10 and about 90 seconds.

Where propane is the reactant, octafluoropropane can be recovered from the effluent by condensing the effluent under sufficiently high pressure and low temperature to condense octafluoropropane, separating the condensate from the uncondensed portion of the effluent, separating an organic phase of the condensate from a hydrogen fluoride phase of the condensate and recovering octafluoropropane from the organic phase of the condensate. The preferred method of recovering octafluoropropane from the organic phase of the condensate is by fractional distillation since, once phase separation has occurred, no compounds boiling close to octafluoropropane will remain in the condensate. It should be appreciated that, if propane itself were present in the effluent, it would also be found in the organic phase of the condensate and would, according to U.S. Patent 3,840,445 complicate purification. In the examples described below having a stoichiometric excess of fluorine, no propane was detected in the effluent and no difficulty should be encountered in separating perfluoropropane from other materials by fractional distillation.

<div align="center">Example 1</div>

Fluorine (0.53 mol/h) and helium (0.25 mol/h) were introduced into a porous tube 15.2 cm (6 inches) in height and 1.9 cm (0.75 inches) in diameter. Propane (0.060 mol/h) was introduced into the base of an annular zone between the outside of the porous member and an outer impervious stainless steel tube. The overall configuration was such that the inner zone of diameter was surrounded by a wall thickness of 0.6 cm (0.25 inches) which in turn was surrounded by an annulus of 0.95 cm (0.375 inches). The pressure inside the porous tube was maintained slightly about the pressure outside the porous tube. Product was withdrawn from the top of the impervious tube, above the upper end of the porous tube. The porous tube was of fused alumina having a porosity (ratio of void space to total volume) of 21%, permeability (mL of air/min. through a piece one inch thick and one square inch in area with a pressure differential of one inch of water) between 12 and 20 and a chemical analysis of 99.55% $Al_2O_3$, 0.07% $SiO_2$, 0.09% $Fe_2O_3$ and 0.25% $K_2O$ and $Na_2O$ (sold by the Norton Company of Worcester, Massachussets as ALUNDUM AN-299A fused alumina, "ALUNDUM" being their registered trademark).

The total product was condensed in a liquid nitrogen chilled trap and analyzed by gas chromatography. Prior to condensation, HF was removed from the effluent by scrubbing with NaF. In a six-hour experiment 122 grams of fluorine and 15.9 grams of propane were fed, for a total of 137.8 grams of reactants fed in. Product recovery was 60 grams HF and 73.4 grams of organic product, for a total of 133.4 grams or a 97 percent mass recovery. Gas chromatography analysis of the organic indicated 2.4 weight percent carbon tetrafluoride, 2.0 percent carbon dioxide, 4.6 percent perfluoroethane, 68.4 percent perfluoropropane, 21.6 percent other three and six carbon fluorinated alkanes and 1.0 percent unknowns. Thus, based solely on the 68.4 percent of the 73.4 grams product, the yield per pass was 73 percent based on propane fed. It should be appreciated that at least a portion of the 21.6 percent was also materials such as partially fluorinated propane which could, if recycled, produce additional perfluoropropane.

## Examples 2-17
### Fluorinations of Propane and Propene in Fused Alumina Porous Tube

Using the porous tube of Example 1, a number of experiments were performed feeding the mol/h of fluorine and mol/h of diluent (either sulfur hexafluoride or nitrogen) into the interior of the porous tube indicated in Table 1 under the heading "Inner Zone". In addition, the amounts of propane or propene and diluent indicated in Table 1 under the heading "Outer Zone" were fed into the annular space around the porous tube. The maximum temperature along the outside of the porous tube was monitored with a thermwell and is indicated in Table 1. Contact times computed from reactant flow rates, reaction temperature and volume of annulus are also indicated in Table 1. Gas chromatographic analysis of the effluent after absorption of HF by NaF was performed and the results compared to control samples so as to calculate a yield of octafluoropropane based on fluorine in cases where less fluorine than the stoichiometric amount was introduced and based on propane or propene fed where less than the stoichiometric amount of propane or propene was introduced. In Table 1 the fluorination ratio or "F. Rat." is shown to indicate the mole ratio of fluorine to propane or propene which should be compared to the stoichiometric amount which is 8 in the case of propane and 7 in the case of propene. The yields reported in Table 1 do not take into account partially fluorinated propanes and propenes which could be converted to additional octafluoropropane if recycled.

### Table 1

Fluorinations of Propane and Propene
In Fused Alumina Porous Tube

| | | Outer Zone | | Inner Zone | | Max. | CT | Yield |
|---|---|---|---|---|---|---|---|---|
| Ex. | F.Rat. | Propane | $SF_6$ | $F_2$ | $SF_6$ | Temp. | Sec. | $C_3F_8$ |
| 2 | 1 | 0.25 | 0.50 | 0.25 | 2.0 | 96°C | 9 | nil |
| 3 | 3 | 0.25 | 0.50 | 0.75 | 2.0 | 210 | 8 | 10 |
| 4 | 1 | 0.25 | 0 | 0.25 | 0.25 | 63 | 37 | 55 |
| 5 | 2 | 0.25 | 0 | 0.50 | 0.25 | 93 | 28 | 47 |
| 6 | 3 | 0.25 | 0 | 0.75 | 0.50 | 252 | 18 | 33 |
| 7 | 9.5 | 0.063 | 0 | 0.60 | 0.50 | 222 | 24 | 71 |
| 7A | 9.5 | 0.063 | 0 | 0.60 | 0.25 | 210 | 30 | 72 |
| | | Propane | $N_2$ | $F_2$ | $N_2$ | | | |
| 8 | 9.5 | 0.063 | 0 | 0.60 | 1.0 | 219 | 17 | 65 |
| 9 | 9.5 | 0.063 | 0 | 0.60 | 0.5 | 232 | 23 | 74 |
| 10 | 9.5 | 0.063 | 0 | 0.60 | 0.25 | 234 | 30 | 73 |
| 11 | 9.5 | 0.063 | 0 | 0.60 | 0 | 233 | 42 | 85 |
| | | Propene | $N_2$ | $F_2$ | $N_2$ | | | |
| 12 | 8.3 | 0.072 | 0 | 0.60 | 1.0 | 230 | 17 | 37 |
| 13 | 8.3 | 0.072 | 0 | 0.60 | 0.5 | 238 | 24 | 44 |
| 14 | 8.3 | 0.072 | 0 | 0.60 | 0.25 | 238 | 30 | 50 |
| 15 | 8.3 or | 0.072 or | 0 | 0.60 | 0 | 235 | 40 | 35 |
| 16 | 7.0 | 0.086 | 0 | 0.60 | 0 | 221 | 30 | 45 |
| | | Propene | $SF_6$ | $F_2$ | $SF_6$ | | | |
| 17 | 8.3 | 0.072 | 0 | 0.60 | 0.25 | 236 | 30 | 40 |

### Examples 18-20

Employing the same apparatus as in Example 1, the amounts of fluorine and nitrogen (in mol/L) shown in Table 2 were introduced into the base of the alumina porous tube and the amounts of acetone and nitrogen (in mol/L) shown in Table 2 were introduced into the base of the annular zone. After absorption of HF in sodium fluoride, the effluent was analyzed by gas chromotography yielding the proportions of hexafluoroacetone, partially fluorinated acetones and byproducts (principally of one or two carbons) indicated in Table 2.

## Table 2
### Fluorination of Acetone In Porous Fused Alumina Tube

| Ex | 18 | 19 | 20 |
|---|---|---|---|
| Fluorination Ratio | 4.6 | 4.6 | 4.6 |
| O.Z. acetone | 0.054 | 0.054 | 0.054 |
| O.Z. nitrogen | 0.10 | 0.10 | 0.10 |
| I.Z. fluorine | 0.25 | 0.25 | 0.25 |
| I.Z. nitrogen | 1.0 | 0.50 | 0.10 |
| Max. Temp. °C | 140 | 148 | 141 |
| GC Analysis (area %): | | | |
| $C_3F_6O$ | 43 | 38 | 40 |
| $C_3F_nH_{6-n}O$ * | 49 | 26 | 28 |
| Byproducts ** | 8 | 36 | 32 |

\* monofluoroacetone thru pentafluoroacetone

\*\* including $CF_4$, $COF_2$, $CF_3COF$

### Example 21 and Comparative Examples 22 and 23

Examples 18-20 were repeated with the feeds and product analysis shown in Table 3, with sulfur hexafluoride as the diluent both for acetone and for fluorine. Similar experiments were conducted as indicated in Table 3 using a porous copper tube (C22) and a nickel tube (C23). The tubes were similar to the alumina tube except that their diameter was 3.8 cm (1.38 inches) instead of 1.9 cm (0.75 inch); their thickness was 0.16 cm (0.063 inch) instead of 0.6 cm (0.25 inch); and their pore sizes were 5 and 10 micrometers, respectively. As shown in Table 3, the yield of hexafluoroacetone was over twice as much and the byproducts less than one quarter as much with the alumina tube compared to the metal tubes.

## Table 3

Fluorination of Acetone In
Different Porous Tubes With
Sulfur Hexafluoride Diluent

| Ex | 21 | C22 | C23 |
|---|---|---|---|
| Tube | Alumina | Copper | Nickel |
| O.Z. Acetone | 0.30 | 0.30 | 0.30 |
| O.Z. $SF_6$ | 0.60 | 0.60 | 0.60 |
| I.Z fluorine | 0.25 | 0.25 | 0.50 |
| I.Z.$SF_6$ | 0.25 | 0.25 | 0.50 |
| GC Analysis (area %): | | | |
| $C_3F_6O$ | 35 | 14 | 14 |
| $C_3F_nH_{6-n}O$ | 50 | 20 | 5 |
| Byproducts | 13 | 66 | 81 |

## Examples 22-26

Examples 2-17 were repeated with the organic feed being normal pentane (Ex 23), cyclopentane (Ex 24), dimethyl ether (Ex 25), trimethylamine ("TMA") (Ex 26), methane (Ex 27) and acetyl fluoride ("AF") (Ex 28). The yields of perfluorinated products $CF_3(CF_2)_3CF_3$, cyclic $(CF_2)_5$, $CF_3OCF_3$, $CF_4$ and $CF_3COF$ are indicated for all Examples except 26. The yields of compounds having less fluorines, but the same carbon, carbon-oxygen or carbon-nitrogen skeleton ("partially fluorinated products") and byproducts (apparently by degradation of the skeleton) are also indicated in Table 4. For trimethylamine, peaks were detected which appeared to be compounds such as $(CF_3)_3N$, $(CF_3)_2(CHF_2)N$, $(CF_3)(CHF_2)_2N$ and $(CHF_2)_3N$. Mass spectral analyses of second and fourth members of the series confirmed their presence.

### Table 4

#### Other Fluorinations With Fused Alumina Porous Tube

| Ex | 23 | 24 | 25 |
|---|---|---|---|
| Organic | n-pentane | c-pentane | dimethyl ether |
| O.Z. Organic | 0.040 | 0.031 | 0.050 |
| O.Z. $N_2$ | 0.10 | 0.10 | nil |
| I.Z. $F_2$ | 0.36 | 0.30 | 0.50 |
| I.Z. $N_2$ | 0.50 | 1.0 | 1.0 |
| Max Temp °C | 210 | 185 | 156 |
| GC Analysis (area %): | | | |
| Perfl. prod. | 54 | 76 | 53 |
| Par. fluorinated prod. | 25 | 10 | 16 |
| Byproducts | 21 | 14 | 31 |

| Ex | 26 | 27 | 28 |
|---|---|---|---|
| Organic | TMA | Methane | AF |
| O.Z. Organic | 0.030 | 0.060 | 0.066 |
| O.Z. $N_2$ | 0.10 | 0.10** | 0.10 |
| I.Z. $F_2$ | 0.30 | 0.25 | 0.21 |
| I.Z. $N_2$ | 0.50 | 0.50** | 2.0 |
| Max. Temp °C | 151 | 136 | 92 |
| GC Analysis (area %) | | | |
| Perfl. prod. | * | 67 | 44 |
| Par. fluorinated prod. | * | 29 | 27 |
| Byproducts | * | 4 | 29 |

* not quantified

**helium diluent used instead of nitrogen

### Examples 29-32

#### Fluorination of Hexafluoropropene in Porous Alumina Tube

The general process of examples 1-17 were repeated with hexafluoropropene ("HFP") as the organic feed. These Examples illustrate the susceptability of double bonds as the sole "fluorinatable carbons" to fluorination by the present process. The feed rates, operating conditions and results are displayed in Table 5. The area % of nitrogen is included in the analysis (diluent was excluded in previous tables).

### Table 5

#### Fluorination of Hexafluoropropene

| Ex | 29 | 30 | 31 | 32 |
|---|---|---|---|---|
| Organic | HFP | HFP | HFP | HFP |
| O.Z. Organic | 0.30 | 0.25 | 0.25 | 0.50 |
| I.Z. $F_2$ | 0.25 | 0.25 | 0.25 | 0.50 |
| I.Z. $N_2$ | 1.0 | 1.0 | 0.50 | 0.25 |
| Max. Temp. | 143°C | 170°C | 184°C | 254°C |
| Contact Time | 18 s | 18.5 s | 28 s | 22 s |
| GC Analysis (Area %): | | | | |
| $N_2$ | 57.8 | 60.8 | 46.4 | 19.3 |
| $CF_4$, $C_2F_6$ | 0.2 | 0.4 | 0.8 | 0.9 |
| $C_3F_6$ | 2.1 | 0 | 0 | 0 |
| $C_3F_8$ | 25.8 | 22.8 | 36.7 | 58.5 |
| $C_6F_{14}$ | 14.1 | 16.0 | 16.1 | 21.3 |
| % Yield $C_3F_8$ ($F_2$ basis) | 80 | 66 | 70 | 67 |

What is claimed is:

1. A method of preparing perhalogenated organic compounds which comprises:

(a) introducing a first gaseous reactant stream comprising fluorine into an elongated inner zone surrounded by an aluminum oxide porous member fused at sufficiently high temperature to be resistant to fluorine corrosion; and

(b) introducing a second gaseous reactant stream outside the aluminum oxide porous member comprising at least one organic compound with at least one fluorinatable carbon selected from the group consisting of carbons covalently bonded to H, Cl or Br and carbons olefinically or acetylenically bonded to other carbons; the pressure in the elongated inner zone being greater than the pressure outside the aluminum oxide porous member.

2. The method of claim 1 wherein the organic compound is an acyclic hydrocarbon of 1-8 carbons having at most one olefinic unsaturation.

3. The method of claim 2 wherein the organic compound is an aliphatic hydrocarbon of 1-8 carbons.

4. The method of claim 1 wherein the organic compound is an acyclic halogenated hydrocarbon having no element other than carbon, hydrogen, chlorine, bromine and fluorine and having at most 2n+1 fluorines where n is the number of carbons.

5. The method of claim 4 wherein the organic compound has at least one hydrogen.

6. The method of any of claims 1 to 4 wherein the organic compound has three carbons and the method includes recovering octafluoropropane from the effluent outside the porous member.

7. The method of claim 6 wherein the organic compound is propane or propene or hexafluoropropene.

8. The method of claim 1 wherein the organic compound is a ketone of 3-6 carbons.

9. The method of any previous claim wherein the overall volume ratio of diluent introduced with the two reactants to total reactants is between 0:1 and about 5:1.

10. The method of claim 8 wherein the organic compound is acetone and the molar ratio of fluorine to acetone is between about 1:1 and about 8:1.

European Patent
Office

**EUROPEAN SEARCH REPORT**

0031519

Application Number

EP 80 10 7797

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| AD | CA - A - 990 738 (ALLIED CHEMICAL CORP.) | | C 07 B 9/00 <br> B 01 J 12/00 <br> 19/24// <br> C 07 C 19/08 <br> 49/167 <br> 23/08 <br> 43/12 <br> 53/48 <br> 85/24 |
| A | DE - C - 651 049 (I.G. FARBENINDUSTRIE) <br><br> ---- | | |

**TECHNICAL FIELDS SEARCHED (Int. Cl.³)**

C 07 B 9/00
C 07 C 17/10
45/63
51/62
85/24
17/04
41/22
43/12
B 01 J 12/00
19/24

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 25-03-1981 | VAN GEYT |

EPO Form 1503.1   06.78